# EUROPEAN PATENT APPLICATION

(11) **EP 4 202 948 A1**
(43) Date of publication of application: **28.06.2023**
(21) Application number: 21217719.0
(22) Date of filing: 24.12.2021
(51) Int. Cl.: G16H 30/40, G16H 50/20, G16H 50/70

(54) **SKIN CONDITION DIAGNOSIS WITH NEURAL NETWORKS**

(71) Applicant: JCM SKN Limited, Poole, Dorset BH14 8UF (GB)
(72) Inventor: CARRIER, Jason, Poole, Dorset, BH14 8UF (GB); HURD, Toby, Poole, Dorset, BH14 8UF (GB); RATNAVEL, Ravi, Poole, Dorset, BH14 8UF (GB); KELMAN, Clive, Poole, Dorset, BH14 8UF (GB); SHAMA, Richard, Poole, Dorset, BH14 8UF (GB)
(74) Representative: Marks & Clerk LLP

(57) **Abstract**

A computer implemented method of diagnosing an affected skin area of a patient using a neural network system, the method comprising: receiving a set of images showing the affected skin area and a plurality of metadata entries regarding the patient and/or symptoms of the affected skin area; processing each of the set of images using an image classification neural network module to produce a respective series of image classification confidence values indicating whether the skin area skin shown in the processed image is affected by each of a list of skin conditions; defining an input array comprising the plurality of metadata entries and each of the series of image classification confidence values produced from the respective images of the set of images; processing the input array using a primary neural network module to produce a series of diagnosis confidence values, each diagnosis confidence value indicating a confidence that the affected skin area corresponds to a skin condition from the list of skin conditions.

## Description

### FIELD

Embodiments described herein relate to neural network systems for diagnosing dermatological conditions and to methods of training such systems.

### BACKGROUND

Skin conditions, such as skin cancers, are typically diagnosed visually. However the accuracy with which medical practitioners, in particular general practitioners rather than specialised dermatologists, are able to do so is imperfect. Computer vision machine learning systems have therefore been applied to diagnose skin conditions from images thereof.

An aim of the present invention is to provide improved systems and tools for accurately diagnosing patients' skin conditions.

### SUMMARY OF THE INVENTION

According to an embodiment, there is provided a computer implemented method of diagnosing an affected skin area of a patient using a neural network system, the method comprising: receiving a set of images showing the affected skin area and a plurality of metadata entries regarding the patient and/or symptoms of the affected skin area; processing each of the set of images using an image classification neural network module to produce a respective series of image classification confidence values indicating whether the skin area skin shown in the processed image is affected by each of a list of skin conditions; defining an input array comprising the plurality of metadata entries and each of the series of image classification confidence values produced from the respective images of the set of images; processing the input array using a primary neural network module to produce a series of diagnosis confidence values, each diagnosis confidence value indicating a confidence that the affected skin area corresponds to a skin condition from the list of skin conditions.

Such a computer implemented method may provide more accurate diagnosis of patients' skin conditions based on images thereof without necessarily requiring relatively high volumes of training data for the image neural network modules comprised by the neural network system.

In some embodiments, the array may be defined by concatenating each of the series of output values produced by processing a respective image of the set of images with each other and with the plurality of metadata entries.

In some embodiments, the image classification neural network module may be trained for less than 10% of the epochs for which the primary neural network module is trained.

In some embodiments, the the primary neural network module 250 is trained using an Adamax optimiser, sparse categorical cross entropy loss, and/or a sigmoid activation function.

In some embodiments, the image classification neural network module may be configured to produce series of confidence values with lower maximum confidence values when processing each image of a set of images than the primary neural network module when processing these series of confidence values and associated metadata entries.

In some embodiments, the image classification neural network module may comprises one or more steps each comprising performing a 1x1 pointwise convolution and subsequently performing a depthwise convolution.

In some embodiments, the set of images may comprise a plurality of images of the affected skin portion taken at different perspectives. Images taken at different perspectives may be taken at different magnifications and/or at different angles.

In some embodiments, the set of images may comprise: a first image of the affected skin area taken at a first angle, a second image of the affected skin area taken at a second angle, the second angle being different to the first angle; and a third image of the affected skin area and a surrounding portion of the patient's body.

In some embodiments, the metadata entries may comprise answers to at least six questions regarding the symptoms of the affected area of skin.

In some embodiments the questions comprise one or more questions regarding the patient's perception of affected skin (such as how the affected skin feels to the patient and/or regarding the visual appearance of the affected skin), one or more questions regarding observed changes to the affected skin portion over time, one or more questions regarding whether the affected skin has expelled any material, one or more questions regarding the external appearance or texture of the affected skin portion, and one or more questions regarding exposure of the patient to the sun. In some embodiments, the questions may comprise one or more questions from each of the categories listed above.

In some embodiments, the answers to the questions comprise whether the affected skin feels hot or warm, whether the patient feels a burning sensation; whether the affected skin has changed, spread or grown at all; whether the affected skin bleeds or has bled in the past; whether the affected skin is painful, or has caused the patient pain; whether the affected skin itches, or has itched; whether the affected skin weeps or oozes; whether the affected skin has scabbed over; whether the affected skin is inflamed; whether the affected skin scars; whether the affected skin is a different colour from the patient's normal skin; whether the affected skin is dry or rough to touch; whether the patient has been exposed to the sun a lot in the past.

In some embodiments, the answers to the questions comprised by the metadata entries may be ternary variables.

In some such embodiments, the answers may each be selected from a positive answer, a negative answer, and a neither positive or negative answer.

In an embodiment, there is provided a method of training a system for diagnosing a skin condition of a patient, the method comprising: constructing a primary training data set comprising a plurality of training examples, each training example comprising an input array, the input array comprising: a plurality of metadata entries regarding a patient with a skin area affected by a skin condition and/or symptoms of such an affected skin area, and a plurality of image classification confidence values, the image classification confidence values indicating whether an area of skin affected by that skin condition in an image is affected by each of a list of skin conditions; and using the primary training data set to train a primary neural network module to process an input array (240) to produce a series of diagnosis confidence values (160) indicating whether an affected skin area is each of the list of skin conditions; wherein the input array comprises a plurality of metadata entries regarding a patient with the affected skin area and/or symptoms of the affected skin area and a plurality of series of image classification confidence values produced from respective images of the patient's affected skin portion using one or more image classification neural network modules.

In some embodiments, the method may comprise: constructing one or more image classification data sets comprising a plurality of images of skin areas affected by of each of the list of skin conditions; and using the one or more image classification data sets to train the one or more image classification neural network modules (230) to process an image of an affected skin portion to produce a respective series of image classification confidence values as to whether the skin portion shown in the processed image is affected by one of the list of skin conditions; wherein each of the series comprised by the array of each of the training examples of the primary training data set is an output of one of the trained one or more image classification neural network modules based on an image of a skin area affected by the respective skin condition.

In some embodiments, the image classification training data set may comprise equal numbers of images of each of the list of skin conditions. The image classification training data set may be constructed from an original data set comprising different numbers of images of each of the list of skin conditions by excluding images of one or more skin conditions and/or duplicating images of one or more skin conditions.

In some embodiments, the primary training data set may comprise a greater number of training examples relating to one or a first subset of the list of skin conditions. The first subset of skin conditions may include melanoma and squamous cell carcinoma.

### BRIEF DESCRIPTION OF THE FIGURES

Fig. 1 shows a patient with a portion of affected skin capturing an image for diagnosis by a neural network system;
Fig. 2 is a diagram of an example of a neural network system for diagnosing skin conditions;
Fig. 3 is a diagram showing an example of the structure of the primary neural network module of a neural network system as shown in Fig. 2;
Fig. 4 is a flowchart of an example of a method of training a neural network system as shown in Fig. 2;
Fig. 5 is a diagram of a user interacting with a neural network system as shown in Fig. 2;
Fig. 6 is a diagram of different use cases for a neural network system as shown in Fig. 2;
Fig. 7 is a diagram of user application and cloud-based diagnostic system comprising a neural network system as shown in Fig. 2; and
Fig. 8 is a diagram of an example computing system for putting an embodiment, as described herein, into effect.

### DETAILED DESCRIPTION OF THE FIGURES

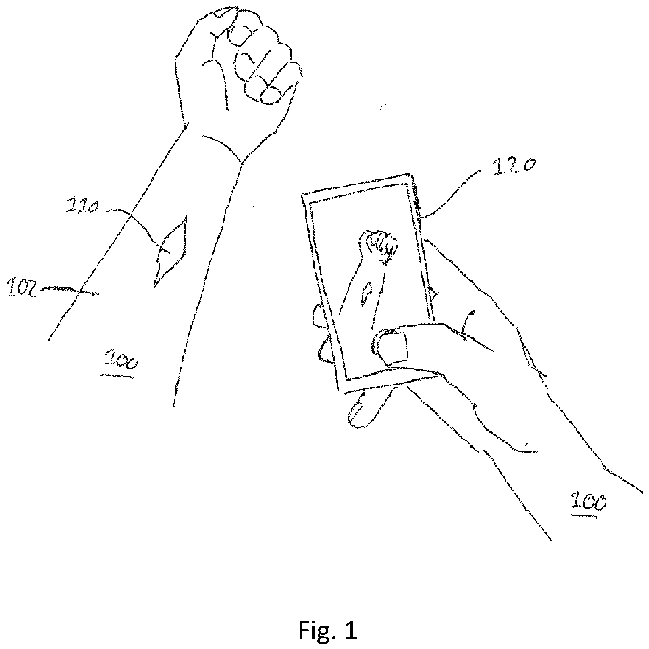

Fig. 1 shows an example of a patient 100 with a portion of affected skin 110 capturing a set of images of the affected skin portion 110, such as a skin lesion, in order to have the affected skin portion 110 diagnosed by a neural network system in accordance with an embodiment.

The neural network system is configured to receive the set of captured images, in conjunction with metadata comprising details of the patient 100 and/or responses to questions relating to the affected skin and/or their perception thereof. These are provided as inputs and to output confidence values as to whether the imaged affected skin portion 110 is each of a number of skin conditions. Such confidence values may be used by the patient, or by a medical professional, in order to diagnose the skin condition affecting the portion of skin 110.

In the illustrated example, the patient 100 captures the images themselves using the camera of a smartphone 120. However, it will be appreciated that in alternative embodiments, some or all of the images may be taken by someone other than the patient 100, for example, by a medical professional such as a general practitioner, and/or may be taken using another device, such a dedicated digital camera, or a webcam connected to another computing device.

While in the illustrated example, the portion of affected skin 110 is on the patient's left forearm 102, affected skin portions, and/or individual parts thereof, on any part of the patient's body may be imaged for diagnosis by the neural network system.

In the illustrated example, the captured images are conventional two-dimensional colour photographs. However, it will be appreciated that in alternative embodiments, some or all of the images may be of other formats, for example, grayscale, three dimensional, ultraviolet, infrared, video, and/or animated.

The patient or other user capturing the images may be instructed or prompted on a manner in to capture the images, for example, by software with which the images are submitted to the neural network

The captured images are submitted to the neural network system, for example, by the patient 100, or by another user such as a medical practitioner. In some embodiments, the neural network system may be remote from the user or a computing device with which they submit the image, such as a smartphone with which they also captured the images as described above. For example, the neural network system may be hosted on a cloud computing service, or a server remote from the user's computing device. In such embodiments, the captured images may be transmitted or uploaded to the neural network system or a host thereof, for example, using an associated web application, or other software.

The patient or other user capturing the images may be instructed or prompted on a manner in which to capture the images, for example, by software with which the images are submitted to the neural network, or with which the images are captured. The instructions or prompt may be to capture the images in specific perspectives (such as one close image straight on, one close image at an angle, and one distant image), and/or in specific lighting (such as without a flash and/or in neutral lighting).

In some embodiments, the patient or other user submitting the images may edit or annotate one, some or all of the submitted images, and/or may be prompted to do so, for example with the associated web application or other software. For example, a user may input, and/or may be prompted to input, a bounding box around the affected skin in one, some or each of the images.

In some embodiments, one, some, or all of the images may be manually edited by the submitting patient or other user and/or automatically edited by the system or software with which it is submitted. For example, the image may be automatically scaled to a specific resolution (such as 300 by 300 pixels) and/or may be cropped (for example to a bounding box as described above).

In alternative embodiments, the neural network system, or a local instance thereof, may hosted directly on a computing device to which the patient 100 or another user has access, such as a smartphone with which the images were captured. In such embodiments, each image may be captured using, or be transferred to, said computing device.

In addition to the captured images, metadata comprising details of the patient 100 and/or answers to questions regarding their symptoms is also submitted to the neural network system as an input.

Some or all of the metadata may be entered into a computing device by a user, such as the patient 100 themselves, or a medical practitioner, for example, using an associated web application, or other software as described above. Alternatively, some or all of the metadata entries, such as details of the patient 100, may be automatically retrieved, for example from a patient database.

The images and/or metadata may be submitted to the neural network system after they are all captured, entered and/or retrieved, or may be submitted as they are captured, entered and/or retrieved.

In some embodiment, the images may be edited automatically, or by a user, before, as, or after they are submitted. For example, the images may be cropped to remove surrounding details, may be resized to a specific resolution, and/or may be filtered to remove noise.

The patient 100 of fig. 1 may submit both a set of three captured images and metadata to an embodiment of neural network hosted on cloud computing service using a web application on the smartphone 120. The web application is configured to enable the patient 100 to upload the captured images and to enter their details and answers for the metadata inputs to the neural network system.

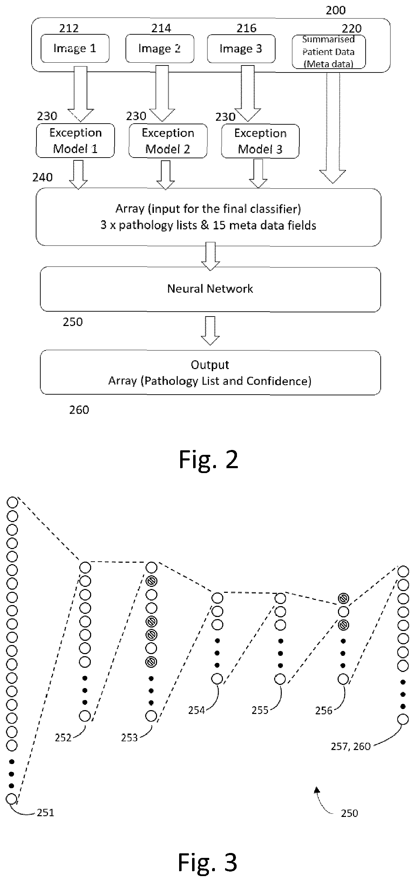

Fig. 2 is a diagram of an example of a neural network system 200 for diagnosing skin conditions. The system 200 is configured to receive inputs 210 comprising a set of images 212, 214, 216 of an affected skin area and a set of associated metadata entries 220 and to output a series of confidence values 260 as to whether the imaged skin condition is each of a list of respective skin conditions.

The neural network system 200 is a modular neural network system comprising a plurality of copies of an image classification neural network module 230 for each classifying one of the set of input images 212, 214, 216 and a primary module 250 that processes an array 240 comprising the outputs of each of the image classification modules 230 and the metadata entries 220 to determine the confidence values 260.

In an embodiment, the system 200 is configured to receive a set comprising a fixed number of images 212, 214, 216 of the affected skin area. In the illustrated neural network system 200, the inputs comprise three different images 212, 214, 216 of an affected skin area. It will be appreciated that different embodiments may use different numbers of images as inputs. Increasing the number of input images generally increases the accuracy with which a neural network system is able to diagnose skin conditions, however the increase in accuracy per image beyond the third is low, so three input images 212, 214, 216 provides an effective balance between accuracy and processing requirements.

In an embodiment, the set of input images 212, 214, 216 show different perspectives of the affected skin area, which may be taken at different angles and/or at different magnifications. For example, at least one of the images may be taken at a different angle to at least one other of the images and/or at least one of the images may be taken at a different magnification to at least one other of the images. The different angles may be separated by at least 20°, or at least 40°, and/or the different magnifications may have a ratio of at least 5:4, or at least 3:2, at least 7:4, or at least 2:1. In some embodiments, the system 200 may be configured to receive inputs 210 that comprise images 212, 214, 216 of a pre-set set of perspectives.

For example, in an embodiment of the neural network system is configured to receive three input images 212, 214, 216, the first image 212 is an image of the affected skin area taken straight on, the second image 214 is an image of the affected skin area taken at a 45° angle, and the third image 216 is a more zoomed-out image of a larger area of the patient's body including the affected skin area. Such a third image 216 may include landmark features of the patient's body which may facilitate in determining the location of the affected skin area.

In the illustrated embodiment, each of the image classification modules 230 is a copy of the same trained neural network module, and each of the input images 212, 214, 216 is classified by one of these copies. This may allow the system to receive any combination of perspectives, although a variety of perspectives may increase the accuracy with which the system is able to diagnose imaged skin conditions. In alternative embodiments, the different modules may be different neural network modules, which may have different structures and/or may be trained separately. For example, each of the image classification modules 230 may be a neural network module that is trained using images of the respective perspective of input images that it classifies.

The metadata entries 220 may comprise details of the patient whose skin is affected, and/or answers to questions regarding the skin condition or symptoms thereof. The metadata entries 220 may comprise answers to at least 10 questions regarding the skin condition or symptoms thereof.

Details of the patient comprised by the metadata entries 220 may comprise the patient's age and/or whether they are male or female.

Questions regarding the skin condition or symptoms thereof may comprise one or more questions regarding how the affected skin feels to the patient themselves, for example: whether the affected skin feels hot or warm, or the patient has a burning sensation; whether the affected skin is painful, or has caused the patient pain; and/or whether the affected skin itches, or has itched. Alternatively, or additionally, the questions may comprise one or more questions regarding any observed changes to the affected skin portion over time, for example: whether the affected area of skin has changed, spread or grown at all; and/or whether the affected skin has scabbed over. Alternatively, or additionally, the questions may comprise one or more questions regarding whether the affected skin has expelled any material, for example: whether the affected skin bleeds or has bled in the past; and/or whether the affected skin weeps or oozes. Alternatively or additionally, the questions may comprise one or more questions regarding the external appearance or texture of the affected skin portion, for example, whether the affected skin is inflamed; whether the affected skin scars; whether the affected skin is a different colour from the patient's normal skin; and/or whether the affected skin is dry or rough to touch; Alternatively, or additionally, the questions may comprise one or more questions regarding exposure of the patient or and/or their skin to the sun, for example, whether the patient is frequently and/or regularly exposed to the sun and/or has been exposed to the sun a lot in the past. In an embodiment, the questions may comprise all of the specific examples listed above. These questions have been found to be particularly pertinent to skin conditions and to allow skin conditions to be diagnosed with a high degree of accuracy by embodiments of a neural network system as described herein.

The answers to some or all of the questions comprised by the metadata entries 220 may be selected from three possible answers for that question (or for each of the questions). The three answers may comprise "yes" or some other positive or affirmative answer; "no" or some other negative answer; and "not applicable", "not answered", "not sure", or some other neither positive nor negative answer. The three answers may be represented as a ternary variable format in which it may be comprised by the intermediate array 240 and/or the inputs 210. For example, the positive answer may be represented as +1, the negative answer may be represented as -1, and the neither positive nor negative answer may be represented as 0. Allowing yes/no questions to be answered with a third neither yes nor no answer may differentiate answers that are relevant and/or in which the answerer is confident from those which are irrelevant, or of which the answerer is unsure. This ternary response input may enable the primary neural network module 250, into which these answers are input, to weight these answers accordingly, preventing erroneous results or training resulting from irrelevant or unsure answers to the questions.

Additionally, the metadata may comprise where on the patient's body the affected skin is located, which may be specified as one of a plurality of locations on a pictogram, list, or other means.

In the illustrated embodiment, the system 200 is configured to receive metadata entries comprising answers to the 12 questions regarding the skin condition or symptoms thereof listed above, the patient's age, whether the patient is male or female, and the location on the patient's body where the affected skin is located, defining 15 metadata entries in total.

The metadata entries may each be feature transformed into numerical values for use by the system. For example, answers to yes/no questions may transformed from "yes", "no", and "not applicable (NA)" to 1, -1, and 0 respectively. Alternatively other method of feature transforming metadata entries into numerical values may be utilised such as one-hot encoding. The metadata entries may be feature transformed into numerical values before they are input into the system and/or by the system after they have been input. A patient's age may be input directly as a numerical value, or may be transformed into one of a series of numerical values corresponding to different age brackets. A location on the patient's body where the affected skin area is located may be transformed into a numerical value corresponding to one of a set of pre-determined locations thereon.

The neural network system 200 comprises a plurality of image classification neural network modules 230 each of which is configured to classify one of the set of input images 110, 112, 114. The illustrated system 100 comprises three copies of such a module 230 for classifying three input images 110, 112, 114 of an affected skin area which may show first to third perspectives thereof.

The image classification neural network modules 230 are each configured to receive a respective image 110, 112, 114 of the set as an input and to output a series of confidence values as to whether the skin condition captured in the image is each of a list of respective skin conditions.

The image classification neural network modules 230 may be a convolutional neural network module. Such convolutional neural network modules may comprise one or more steps in which a a 1x1 pointwise convolution is performed, followed by a depthwise convolution (i.e. a spatial convolution performed independently over each channel of an input). Such steps are a variation on depthwise separable convolutions (in which a depthwise convolution is performed first, followed by a 1x1 pointwise convolution). Such steps may arranged in a linear stack, and/or may have linear residual connections around them. For example, the image classification neural network modules 230 may be Xception modules as described in the paper "Xception: Deep Learning with Depthwise Separable Convolutions" by Chollet F. Such modules may entirely decouple the mapping of cross-channels correlations and spatial correlations in the feature maps of convolutional neural networks, and have been found to provide greater accuracy than other image classification modules (such as those of the Inception architecture family of convolutional neural networks developed by Szegedy C et al.).

In some embodiments, the image classification neural network modules 230 may be trained from a base model using weights from ImageNet with average pooling. The image classification neural network modules 230 may be trained using Softmax activation, .h5 format, sparse categorical cross entropy from logits loss, and/or a list of skin conditions as classes.

The listed skin conditions may comprise: acne, actinic keratosis (AK), angioma or haemanioma, basal cell carcinoma (BCC), Behçet's disease (BD), benign nevus (BN), bowen's disease, eczema or dermatitis, folliculitis, fungal skin infection, hidradenitis suppurativa, keloid, lentigo, lichen planus, melanoma (mel), prurigo, psoriasis, squamous cell carcinoma (SCC), sebhorrheic keratosis (SK), skin pigmentation, urticaria, verruca ,and/or warts.

The confidence values output the by the image classification neural networks for each of the list of skin conditions may be normalised, for example such that they sum to 1 if they are decimal fraction values, or to 100% if they are percentages. The table below shows an example of the confidence values output by three copies of an image classification module 230 on three different perspective images of an affected skin portion. Based on these confidence values and associated metadata entries, the primary module output a 96.3% confidence that the imaged skin condition is basal cell carcinoma.

**Table I: Example confidence values output by image classification modules**

| Skin Condition | Confidence shown in 1^{st} Image | Confidence shown in 2^{nd} Image | Confidence shown in 3^{rd} Image |
|---|---|---|---|
| Acne | 7.96% | 12.43% | 0.91% |
| Actinic Keratosis | 0.01% | 0.00% | 0.02% |
| Basal Cell Carcinoma | 38.44% | 52.38% | 5.61% |
| Bowen's Disease | 2.64% | 0.02% | 0.07% |
| Benign Nevus | 17.17% | 0.91% | 80.95% |
| Eczema or Dermatitis | 0.19% | 0.01% | 1.13% |
| Folliculitis | 0.01% | 0.00% | 0.04% |
| Fungal Skin Infection | 0.04% | 0.00% | 0.14% |
| Hidradenitis Suppurativa | 0.03% | 0.00% | 0.00% |
| Keloid | 0.05% | 0.00% | 0.01% |
| Lentigo | 0.03% | 0.12% | 0.27% |
| Lichen Planus | 0.01% | 0.00% | 0.01% |
| Melanoma | 0.13% | 0.03% | 0.00% |
| Prurigo | 0.04% | 0.00% | 0.00% |
| Psoriasis | 2.88% | 0.33% | 0.02% |
| Squamous Cell Carcinoma | 0.01% | 0.00% | 0.17% |
| Seborrheic Keratosis | 29.87% | 33.45% | 9.54% |
| Skin Pigmentation | 0.00% | 0.00% | 0.00% |
| Urticaria | 0.00% | 0.00% | 0.01% |
| Verruca | 0.48% | 0.31% | 1.11% |
| Warts | 0.01% | 0.00% | 0.01% |

The image classification neural network modules 230 may be trained using a relatively low number of epochs. This may prevent these modules 230 from overfitting to training images and provide relatively low levels of certainty to the confidence values output by the image classification neural network modules 230. In the diagnosis of skin conditions, it may be advantageous for the image classification modules 230 to classify the images as showing given skin conditions with relatively low confidence values and/or with non-negligible confidence values spread over a number of potential skin conditions, so as to ensure all potential conditions are adequately considered. The primary neural network module 250 may then produce a more confident diagnosis based on the confidence values output by the image classification modules 230 and the metadata 220. The image classification neural network may be trained to classify the images to classify the images as showing given skin conditions with relatively high tolerances in the accuracy of their confidence values. Training the mage classification modules 230 with such relatively low numbers of epochs has been found to improve the accuracy with which the system 100 as a whole is able to diagnose skin conditions, while also facilitating training of the system 200. The image classification neural network modules may be trained until they have accuracies of between 65% and 90%, or between 70% and 85%, on their training data set.

For example, the image classification neural network modules 230 may be trained using, less than 40 epochs, less than 25 epochs, and/or less than 15 epochs. The image classification neural network modules 230 may be trained using 10% or less of the number of the epochs with which the primary module 250 is trained. For example, the image classification neural network modules 230 may be trained using 10 epochs, while the primary module 250 may be trained using 200 epochs.

The confidence values output by each of the image classification modules 230 are each fed into an intermediate array 240 that defines the inputs to the primary module 250. The metadata entries are also fed into this array 240. The intermediate array 240 may be constructed by concatenating the outputs of each of the image classification modules 230 and the metadata entries 220.

The intermediate array 240 comprises the confidence values output by each of the image classification modules 230 separately from each other. In an embodiment, these output classification values are maintained separately and all input into the primary module 250, rather than being averaged. The separate inputting of the outputs from the image classification modules maintains information from the initial image classification. This is useful, for example, in situations where one of the image classification modules 230 outputs a significantly different confidence value for a given skin condition to the other image classification modules 230. Additionally, this configuration of the intermediate array 240 may allow the primary module 250 to differentiate between the classifications of the different perspective images, for example to allow the primary module to learn to apply different weightings thereto.

The primary module 250 is configured to receive the intermediate array as an input and to output a series of confidence values 260 as to whether the patient's skin condition is each of a list of respective skin conditions.

In an embodiment the primary module 250 comprises a plurality of dense layers and one or more dropout layers. Fig. 3 shows an example of a primary module 250 for a neural network system comprising the following layers in the following order: a first dense layer 251 with relu activation; a second dense layer 252 with relu activation and half the outputs of the first dense layer 251; a first dropout layer 253 with 0.5 value; a third dense layer 254 with relu activation and half in the outputs of the preceding second dense layer 252; a fourth dense layer 255 with relu activation and same number of outputs of the preceding third dense layer 254; a second dropout layer 256 with 0.1 value; and a final fifth dense layer 257 with sigmoid or linear activation with outputs equal to the number of possible skin conditions. The final fifth dense layer defining the series of confidence values 260.

In alternative embodiments, the primary module 250 may have different structures. For example the primary module 250 may be a convolutional neural network.

The primary neural network module 250 may be trained may using an Adamax optimiser, sparse categorical cross entropy loss, a sigmoid activation function, and/or a list of skin conditions classes as classes.

The primary module 250 may be trained using a relatively large number of epochs. For example, the primary module may be trained using more than 100 epochs, and/or more than 150 epochs. The primary module may be trained using more than 10 times the number of epochs with which the image classification neural network modules 230 are trained. For example, the image classification neural network modules 230 may be trained using 10 epochs, while the primary module 250 may be trained using 200 epochs.

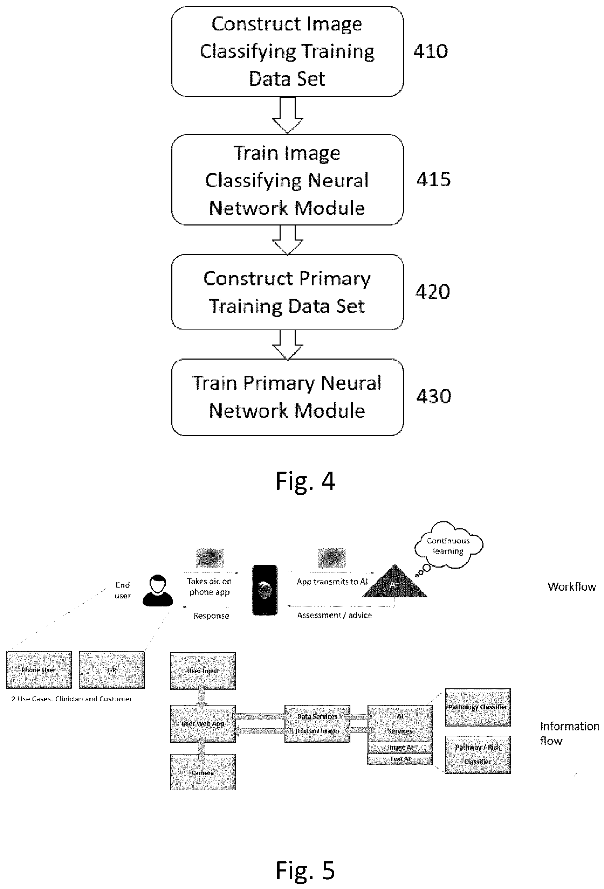

Fig. 4 shows an example of an embodiment of a method 400 of training a neural network system such as the neural network system 100 shown in Fig. 2.

The method comprises constructing 410 a image classifying training data set for training the image classification neural network module 230. The image classifying training data set comprises images of portions of skin affected by each of the skin conditions of the classes of the image classification neural network module 230.

The image classifying training data set may comprise images in each of the perspectives that the system 200 is configured to receive in its inputs 210, in order to train a single image classification module 230 to classify images in all perspectives. In alternative embodiments, the image classifying training dataset may consist of subsets of images in each of the perspectives for independently training different modules 230 to classify each of the perspectives.

The image classifying training data set (and/or each subset thereof) may comprise equal numbers of images of portions of skin affected by each of the list of skin conditions. Alternatively, the image classifying training data set (and/or each subset thereof) may comprise approximately equal numbers of images of portions of skin affected by each of the skin conditions (for example, having numbers of images of each skin condition within 25%, or within 10%, of each other, or of a default number).

In such embodiments, the image classifying training data set (and/or each subset thereof) may be constructed from another original data set comprising different numbers of images of each pathology by excluding images of skin conditions that are over-represented in the original data set, and/or by duplicating copies of images of skin conditions that are under-represented in the original data (optionally, rotating or otherwise transforming such duplicate copies). The table below shows an example of the construction of an image classifying training data set comprising 15,000 images of each skin condition from an original data set using such methods.

**Table II: Construction of an example image classifying training data set**

| Skin Condition | Number of Images in Original Data Set | Number of Images in Image Classifying Training Data Set |
|---|---|---|
| Acne | 94,680 | 15,000 |
| Actinic Keratosis | 41,506 | 15,000 |
| Angioma or Haemangioma | 4,872 | 15,000 |
| Basal Cell Carcinoma | 68,810 | 15,000 |
| Benign Nevus | 62,394 | 15,000 |
| Bowen's Disease | 6,190 | 15,000 |
| Eczema or Dermatitis | 145,068 | 15,000 |
| Folliculitis | 5,815 | 15,000 |
| Fungal Skin Infection | 10,020 | 15,000 |
| Keloid | 6,845 | 15,000 |
| Lentigo | 4,656 | 15,000 |
| Lichen Planus | 11,323 | 15,000 |
| Melanoma | 3,662 | 15,000 |
| Prurigo | 3,549 | 15,000 |
| Psoriasis | 55,500 | 15,000 |
| Squamous Cell Carcinoma | 4,619 | 15,000 |
| Seborrheic Keratosis | 68,623 | 15,000 |
| Skin Pigmentation | 19,186 | 15,000 |
| Urticaria | 6,788 | 15,000 |
| Verruca | 3,581 | 15,000 |
| Warts | 11,989 | 15,000 |

Adjusting the number of images in the image classifying training data set in this manner may ensure that the trained image classifying module 230 is able to more accurately identify and categorise less common skin conditions of which fewer images are available to train the image classifying module 230.

After the image classifying training data set is constructed, the image classifying neural network module 230 is trained 415 using the image classifying training data set. In alternative embodiments, a plurality of image classifying neural network modules 230 may be trained 415 using subsets thereof of different perspective images. The image classifying neural network module or modules 230 may be trained using relatively low numbers of epochs and/or using any of the other features described above.

Training an image classifying neural network module 230 using the image classifying training data set (or subset thereof) may comprise splitting the data set or subset into a training and test datasets, for example using an 80:20 split.

In alternative embodiments, the first and second steps 410, 420 may be omitted and one or more pre-trained image classifying modules may be used instead.

The method comprises constructing 420 a primary training data set for use in training the primary module 250 of the neural network system 200. The primary training data set comprises a plurality of training examples, each of which comprises an array with a plurality of metadata entries regarding a patient with an effected skin area and/or symptoms of such an affected skin area, and a plurality of series of image classification confidence values as to whether a respective area of skin is affected by each of a list of skin conditions.

Each of the plurality of series may be the output of an image classifying neural network module (230), which may be a module as trained in the second step 420 described above, based on an image of a respective affected skin area.

In some such embodiments, one, some, or each of the plurality of series comprised by the array of one, some, or each of the training examples may be based on the same affected skin portion of the same patient. The plurality of metadata entries comprised by such an array may be regarding that patient and/or their symptoms.

However, in some situations, insufficient images may be available to construct a primary training data set in which each of the plurality of series comprised by the array of one, some, or each of the training examples may be based on the same affected skin portion of the same patient. Therefore, the plurality of series comprised by the array of one, some, or each of the training examples may be based on images of different affected skin portions, which may be on different patients, but which are affected by the same skin condition. The plurality of metadata entries comprised by such an array may be regarding any patient affected by that skin condition and/or their symptoms.

In some embodiments, each of the plurality of series comprised by the array of one, some, or each of the training examples may be based on three different perspectives of an affected skin portion, for example, comprising confidence values derived from a straight-on image, an angled image, and a zoomed out image showing surroundings of the affected skin portion.

In some embodiments, the primary training data set may be constructed from an original data set comprising different numbers of images of each skin condition (and/or metadata entries relating to patients with each skin condition) by excluding images (and/or metadata entries) of skin conditions that are over-represented in the original data set, and/or that are that are less dangerous (for example, that are benign) and/or by duplicating copies of images of skin conditions that are under-represented in the original data and/or that are more dangerous (for example, that are not benign), optionally, rotating or otherwise transforming such copies. Over-representing more dangerous skin conditions may make the trained module more sensitive to such skin conditions.

The second training data set may comprise a greater number of (for example, twice as many) training examples of one or more more dangerous skin conditions than training examples of the remaining conditions. For example, the table below shows an example of the construction of a second training data set comprising 5,000 training examples for each skin condition except melanoma and squamous cell carcinoma (SCC), which each have 15,000 training examples. Each training example comprises, three series of image classification confidence values indicating whether a respective area of skin in a respective one of the images is affected by each of a list of skin conditions, along with a plurality of metadata entries regarding a patient with an skin area affected by said skin conditions and/or symptoms of such an effected skin area. Melanoma and SCC are relatively dangerous skin cancers (for example, being more dangerous than Basal Cell Carcinoma.

**Table III: Construction of an example Primary Training Data Set**

| Skin Condition | Number of Images in Original Data Set | Number of Three-Series Training Examples in Primary Training Data Set |
|---|---|---|
| Acne | 94,680 | 5,000 |
| Actinic Keratosis | 41,506 | 5,000 |
| Angioma or Haemangioma | 4,872 | 5,000 |
| Basal Cell Carcinoma | 68,810 | 5,000 |
| Benign Nevus | 62,394 | 5,000 |
| Bowen's Disease | 6,190 | 5,000 |
| Eczema or Dermatitis | 145,068 | 5,000 |
| Folliculitis | 5,815 | 5,000 |
| Fungal Skin Infection | 10,020 | 5,000 |
| Keloid | 6,845 | 5,000 |
| Lentigo | 4,656 | 5,000 |
| Lichen Planus | 11,323 | 5,000 |
| Melanoma | 3,662 | 15,000 |
| Prurigo | 3,549 | 5,000 |
| Psoriasis | 55,500 | 5,000 |
| Squamous Cell Carcinoma | 4,619 | 15,000 |
| Seborrheic Keratosis | 68,623 | 5,000 |
| Skin Pigmentation | 19,186 | 5,000 |
| Urticaria | 6,788 | 5,000 |
| Verruca | 3,581 | 5,000 |
| Warts | 11,989 | 5,000 |

After the primary training data set is constructed 420, the primary module 250 is trained 430 using the primary training data set. The primary neural network module 250 may be trained using a relatively high number of epochs and/or any other optional features described above.

It will be appreciated that in alternative embodiments, neural network systems 200 as described above with reference to Fig. 2 may be trained using other methods.

Fig. 5 is a diagram of an example of a user 510 interacting with a neural network system 530 as described above using a smartphone 520. As described above with reference to fig. 1, the user 510, who may be a patient, or a general practitioner captures a set of images of an affected skin app using a camera 522 of the smartphone and then provides the images and enters patient details and answers to questions regarding symptoms using a web app 524 on the smartphone. The images and metadata entries are transmitted to the remote neural network system 530 which calculates confidence values that the imaged affected skin portion is each of a list of skin conditions. These confidence values, or a diagnosis or advice derived therefrom are then transmitted to the smartphone 520 to be viewed by the user 510.

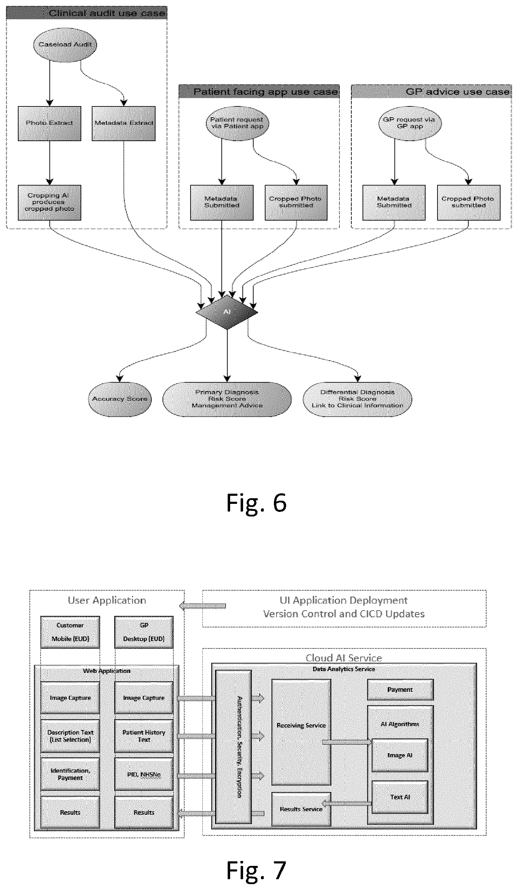

Fig. 6 is a diagram of different use cases for a neural network system 100 as described above. Three different use cases are presented, a clinical audit use case, a patient facing application use case, and a general practitioner advice case.

Fig. 7 is a diagram of user application and cloud-based diagnostic system comprising a neural network system 100 as described above.

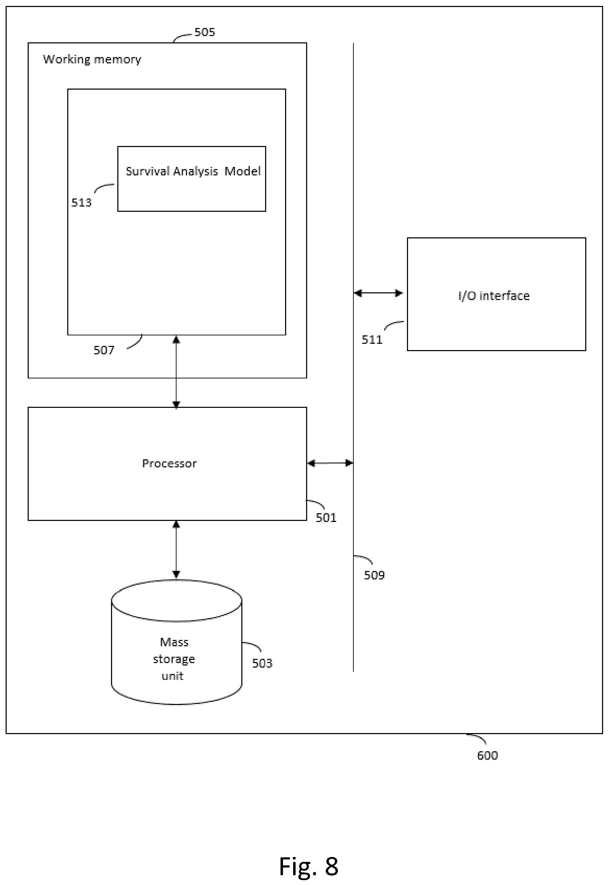

While it will be appreciated that the above embodiments are applicable to any computing system, an example computing system is illustrated in Fig. 8, which provides means capable of putting an embodiment, as described herein, into effect. As illustrated, the computing system 600 comprises a processor 601 coupled to a mass storage unit 603 and accessing a working memory 605. As illustrated, a diagnostic model 613 is represented as software products stored in working memory 605. However, it will be appreciated that elements of the diagnostic model 613, may, for convenience, be stored in the mass storage unit 603.

Usual procedures for the loading of software into memory and the storage of data in the mass storage unit 603 apply. The processor 601 also accesses, via bus 609, an input/output interface 611 that is configured to receive data from and output data to an external system (e.g. an external network or a user input or output device). The input/output interface 611 may be a single component or may be divided into a separate input interface and a separate output interface.

Thus execution of the diagnostic model 613 by the processor 601 will cause embodiments as described herein to be implemented.

The diagnostic model 613 can be embedded in original equipment, or can be provided, as a whole or in part, after manufacture. For instance, the diagnostic model 613 can be introduced, as a whole, as a computer program product, which may be in the form of a download, or to be introduced via a computer program storage medium, such as an optical disk. Alternatively, modifications to existing diagnostic model software can be made by an update, or plug-in, to provide features of the above described embodiment.

The computing system 600 may be an end-user system that receives inputs from a user (e.g. via a keyboard, camera etc) and retrieves a response to a query using diagnostic model 613 adapted to produce the user query in a suitable form. Alternatively, the system may be a server that receives input over a network and determines a response. Either way, the use of the diagnostic model 613 may be used to determine an appropriate diagnosis for a skin condition, as discussed with regard to Figures 1 to 7.

Implementations of the subject matter and the operations described in this specification can be realized in digital electronic circuitry, or in computer software, firmware, or hardware, including the structures disclosed in this specification and their structural equivalents, or in combinations of one or more of them. Implementations of the subject matter described in this specification can be realized using one or more computer programs, i.e., one or more modules of computer program instructions, encoded on computer storage medium for execution by, or to control the operation of, data processing apparatus. Alternatively or in addition, the program instructions can be encoded on an artificially-generated propagated signal, e.g., a machine-generated electrical, optical, or electromagnetic signal that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

While certain embodiments have been described, these embodiments have been presented by way of example only, and are not intended to limit the scope of the inventions. Indeed the novel methods and systems described herein may be embodied in a variety of other forms; furthermore, various omissions, substitutions and changes in the form of methods and systems described herein may be made without departing from the spirit of the inventions. The accompanying claims and their equivalents are intended to cover such forms of modifications as would fall within the scope and spirit of the inventions.

## Claims

1. A computer implemented method of diagnosing an affected skin area (110) of a patient (100) using a neural network system (200), the method comprising:
receiving a set of images (212, 214, 216) showing the affected skin area and a plurality of metadata entries (220) regarding the patient and/or symptoms of the affected skin area;
processing each of the set of images using an image classification neural network module (230) to produce a respective series of image classification confidence values indicating whether the skin area skin shown in the processed image is affected by each of a list of skin conditions;
defining an input array (240) comprising the plurality of metadata entries and each of the series of image classification confidence values produced from the respective images of the set of images; and
processing the input array using a primary neural network module (150) to produce a series of diagnosis confidence values (160), each diagnosis confidence value indicating a confidence that the affected skin area corresponds to a skin condition from the list of skin conditions.

2. A method according to claim 1, wherein the input array is defined by concatenating each of the series of output values produced by processing a respective image of the set of images with each other and with the plurality of metadata entries.

3. A method according to claim 1 or claim 2, wherein the image classification neural network module (230) is trained for less than 10% of the epochs for which the primary neural network module (250) is trained.

4. A method according to any preceding claim, wherein the primary neural network module (250) is trained using an Adamax optimiser, sparse categorical cross entropy loss, and a sigmoid activation function.

5. A method according to any preceding claim, wherein the image classification neural network module (230) is configured to produce series of diagnosis confidence values with lower maximum confidence values when processing each image of a set of images than the primary neural network module (160) when processing these series of confidence values and associated metadata entries.

6. A method according to any preceding claim, wherein the image classification neural network module (230) comprises one or more steps each comprising performing a 1x1 pointwise convolution and subsequently performing a depthwise convolution.

7. A method according to any preceding claim wherein the set of images comprises a plurality of images of the affected skin portion taken at different perspectives.

8. A method according to claim 7, wherein the set of images comprise:
a first image of the affected skin area taken at a first angle;
a second image of the affected skin area taken at a different second angle; and
a third image of the affected skin area and a surrounding portion of the patient's body.

9. A method according to any preceding claim, wherein the metadata entries (120) comprise answers to at least six questions regarding the symptoms of the affected area of skin.

10. A method according to claim 9, wherein the questions comprise at least one question selected from a plurality of the following groups:
(i) questions regarding how the affected skin feels to the patient;
(ii) questions regarding observed changes to the affected skin portion over time;
(iii) questions regarding whether the affected skin has expelled any material;
(iv) questions regarding the external appearance or texture of the affected skin portion; and
(v) questions regarding exposure of the patient to the sun.

11. A method according to claim 9 or claim 10, wherein the questions related to one or more selected from:
(i) whether the affected skin feels hot or warm;
(ii) whether the patient feelsa burning sensation;
(iii) whether the affected skin has changed, spread or grown at all;
(iv) whether the affected skin bleeds or has bled in the past;
(v) whether the affected skin is painful, or has caused the patient pain;
(vi) whether the affected skin itches, or has itched;
(vii) whether the affected skin weeps or oozes;
(viii) whether the affected skin has scabbed over;
(ix) whether the affected skin is inflamed;
(x) whether the affected skin scars;
(xi) whether the affected skin is a different colour from the patient's normal skin;
(xii) whether the affected skin is dry or rough to touch; and
(xiii) whether the patient has been exposed to the sun a lot in the past.

12. A method according to any of claims 9 to 11, wherein the answers to the questions comprised by the metadata entries are ternary variable.

13. A method according to claim 12 wherein each answer is selected from a positive answer, a negative answer, and a neither positive or negative answer.

14. A method of training a system for diagnosing a skin condition of a patient (120), the method comprising:
constructing a primary training data set comprising a plurality of training examples, each training example comprising an input array, the input array comprising:
a plurality of metadata entries regarding a patient with a skin area affected by a skin condition and/or symptoms of such an affected skin area, and
a plurality of image classification confidence values, the image classification confidence values indicating whether an area of skin affected by that skin condition in an image is affected by each of a list of skin conditions; and
using the primary training data set to train a primary neural network module to process an input array (240) to produce a series of diagnosis confidence values (160) indicating whether an affected skin area is each of the list of skin conditions;
wherein the input array comprises a plurality of metadata entries regarding a patient with the affected skin area and/or symptoms of the affected skin area and a plurality of series of image classification confidence values produced from respective images of the patient's affected skin portion using one or more image classification neural network modules.

15. A method according to claim 14, the method comprising:
constructing one or more image classification data sets comprising a plurality of images (112, 114, 116) of skin areas affected by of each of the list of skin conditions; and
using the one or more image classification data sets to train the one or more image classification neural network modules (230) to process an image of an affected skin portion to produce a respective series of image classification confidence values, each confidence value indicating whether the skin portion shown in the processed image is affected by a condition from the list of skin conditions;
wherein each of the series comprised by the input array of each of the training examples of the primary training data set is an output of one of the trained one or more image classification neural network modules based on an image of a skin area affected by the respective skin condition.

16. A method according to claim 15 wherein the image classification training data set comprises equal numbers of images of each of the list of skin conditions.

17. A method according to claim 16 wherein the image classification training data set is constructed from an original data set comprising different numbers of images of each of the list of skin conditions by excluding images of one or more skin conditions and/or duplicating images of one or more skin conditions.

18. A method according to any of claims 14 to 17, wherein the primary training data set comprises a greater number of training examples relating to one of a first subset of the list of skin conditions.

19. A method according to claim 18 wherein the first subset of skin conditions include melanoma and squamous cell carcinoma.
